# EUROPEAN PATENT SPECIFICATION

(11) **EP 3 281 578 B1**
(45) Date of publication and mention of the grant of the patent: **22.05.2024**
(21) Application number: 17779337.9
(22) Date of filing: 04.04.2017
(51) Int. Cl.: A61B 5/00, A61B 18/20, A61N 5/06, A61N 5/067, A61B 17/00, A61B 18/00

(54) **SYSTEM FOR DIAGNOSING DISEASES ON BASIS OF LASER**
SYSTEM ZUR DIAGNOSE VON ERKRANKUNGEN BASIEREND AUF LASER
SYSTÈME DE DIAGNOSTIC DE MALADIES BASÉ SUR UN LASER

(30) Priority: 04.04.2016 KR 20160041316; 06.04.2016 KR 20160041993; 08.09.2016 KR 20160115675
(43) Date of publication of application: 14.02.2018
(73) Proprietor: Speclipse, Inc., Seoul 06247 (KR)
(72) Inventor: PYUN, Sung Hyun, Seoul 06375 (KR); MIN, Wan Ki, Yongin-si Gyeonggi-do 16827 (KR)
(74) Representative: Grünecker Patent- und Rechtsanwälte PartG mbB
(86) International application number: PCT/KR2017/003701
(87) International publication number: WO 2017/176037

(56) References cited:
- WO-A1-2009/137740
- JP-A- H06 222
- JP-A- 2005 192 944
- KR-A- 970 061 214
- KR-A- 20020 018 254
- KR-A- 20090 104 224
- US-A- 5 280 788
- US-A- 5 720 894
- US-A1- 2005 200 843
- US-A1- 2011 300 504
- US-A1- 2015 230 863
- US-A1- 2016 062 009

## Description

### [Technical Field]

The present invention relates to a laser-based disease diagnosis system.

### [Background Art]

Related-art skin toning devices, skin peeling devices, or laser surgery devices perform operations for beauty or medical care using a laser. However, these devices are not used for any other purpose except for the purpose of medical or beauty care.
WO 2009/137740 A1 relates to medical devices for diagnosing and treating anomalous tissue and methods of use. The medical device can comprise an energy source configured to emit at least an excitation beam and a therapeutic beam, a probe coupled to the energy source and configured to propagate the excitation and therapeutic beams with the beams capable of contact with the tissue, a sensor coupled to the probe that detects at least one predefined attribute of radiation emanating from the tissue when the tissue is subjected to the excitation beam and a controller coupled to the energy source and the sensor and programmed to selectively alternatively actuate the energy source to emit the excitation beam and the therapeutic beam in response to the detection of the at least one predefined attribute by the sensor.
US 5,280,788 A relates to systems and methods employed in the use of spectroscopy in the diagnosis of tissue. More particularly, it relates to the use of surface contact and needle probe devices having one or more optical fibers used to direct laser radiation onto tissue to be diagnosed, collecting radiation subsequently emitted by the tissue and delivering that radiation to a system for spectral analysis.
US 5,720,894 A relates to a method and apparatus for fast, efficient, precise and damage-free biological tissue removal using an ultrashort pulse duration laser system operating at high pulse repetition rates. The duration of each laser pulse is on the order of about 1 fs to less than 50 ps such that energy deposition is localized in a small depth and occurs before significant hydrodynamic motion and thermal conduction, leading to collateral damage, can take place. The depth of material removed per pulse is on the order of about 1 micrometer, and the minimal thermal and mechanical effects associated with this ablation method allows for high repetition rate operation, in the region 10 to over 1000 Hertz, which, in turn, achieves high material removal rates. The input laser energy per ablated volume of tissue is small, and the energy density required to ablate material decreases with decreasing pulse width. The ablation threshold and ablation rate are only weakly dependent on tissue type and condition, allowing for maximum flexibility of use in various biological tissue removal applications. The use of a chirpedpulse amplified Titanium-doped sapphire laser is disclosed as the source.
US 2005/200843 A1 relates to an apparatus, a system and a method for detecting the presence or absence of trace elements in a biological sample using Laser-Induced Breakdown Spectroscopy. The trace elements are used to develop a signature profile which is analyzed directly or compared with the known profile of a standard. The apparatus, system and method are used to detect malignant cancer cells in vivo.
US 2016/062009 A1 relates to an interface between two different optical materials which comprise a stack of thin film layers that manage light incident on that interface. One of the optical materials can have a first composition and a first refractive index, while the other optical material can have a second composition and a second refractive index. The stack can comprise thin film layers of the first optical material interleaved between thin film layers of the second optical material. The layers of the stack can be configured to provide the stack with an aggregate composition of at least one of the optical materials that progressively varies from one end of the stack to the other end. To provide the progressive variation in composition, the layers of one of the optical materials can have a progressively increased thickness across the stack, or can progressively increase in number, for example.
US 2015/230863 A1 relates to a dermatological treatment and analysis system that includes a handheld treatment device having a handheld body, a treatment radiation source that delivers a dermatological treatment to the skin, and skin sensor(s) configured to generate signals indicative of one or more skin properties. A wireless transmitter is integrated in the handheld treatment device or in a docking/charging station that receives the handheld treatment device. The wireless transmitter is configured to receive skin-related data comprising the signals from the at least one skin sensor and/or information derived from such signals, and to wirelessly transmit the received skin-related data for analysis of the skin-related data by a remote data analysis system, which may analyze the received skin-related data to generate skin analysis data, and communicate the skin analysis data as feedback to the user of the handheld treatment device, e.g., via a website or application hosted on an internet-connected device of the user.

### [Detailed Description of the Present Disclosure]

### [Technical Objects]

It is the object of the present invention to provide an improved laser-based disease diagnosis system.

This object is solved by the subject matter of the independent claims.

Preferred embodiments are defined in the dependent claims.

According to one embodiment of the present invention, there is provided a laser-based disease diagnosis system.

According to one embodiment of the present invention, there is provided a laser-based disease diagnosis system which is capable of measuring a skin age.

### [Technical Solving Means]

According to one embodiment of the present invention, there is provided a laser-based disease diagnosis system including: a spectrometer configured to measure a spectrum of light which is generated when body tissue is irradiated with a laser for beauty or medical care; a spectrum data comparison unit configured to compare the spectrum of the generated light measured by the spectrometer and a disease diagnosis reference spectrum data DB; and a disease diagnosis unit configured to determine whether there exists a disease in the body tissue based on a result of comparing by the spectrum data comparison unit.

### [Advantageous Effect]

According to one or more embodiments of the present invention, the system can diagnose a disease at the same time as performing an operation for beauty or medical care like skin toning or peeling or laser surgery.

According to one or more embodiments of the present invention, the system can diagnose a disease using an aesthetic or medical laser irradiation device like a skin toning device, a skin peeling device, or a laser surgery device.

According to one or more embodiments of the present invention, the system can diagnose a disease and also measure a skin age using an aesthetic or medical laser irradiation device like a skin toning device, a skin peeling device, or a laser surgery device.

According to one or more embodiments of the present invention, the system can diagnose a disease and measure a skin age, as well as performing operations for beauty or medical care, by adding a simple device without changing the configuration of a related-art laser irradiation device.

### [Brief Description of the Drawings]

FIG. 1 is a view to illustrate a laser-based disease diagnosis system according to one example illustrating the present invention;
FIG. 2 is a view to illustrate an analysis unit according to one embodiment;
FIG. 3 is a view to illustrate an analysis unit according to another embodiment;
FIGs. 4 and 5 are views to illustrate a laser-based disease diagnosis system according to another example not according to the present invention;
FIGs. 6 and 7 are views to illustrate a probe according to one embodiment of the present invention;
FIG. 8 is a view to illustrate a laser-based disease diagnosis system according to another embodiment; and
FIG. 9 is a view to illustrate a laser-based disease diagnosis system according to another embodiment.

### [Explanation of Signs]

1: body tissue
20, 120: analysis device
30, 130: laser irradiation device
21, 121, 221, 421, 521: analysis unit
222: diagnosis server
224: diagnosis terminal
23: probe
31: laser main body
33: handpiece
40, 440, 540: display
111, 211, 421, 521: spectrometer
112, 212, 218, 412, 512: computer processor
114, 214, 216, 414, 514: HW/SW resources
113, 213, 413, 513: disease diagnosis unit
115, 215, 415, 515: disease diagnosis reference spectrum DB storage unit
117, 217, 517: skin age measurement unit
119, 219, 519: skin age measurement reference spectrum DB storage unit;
407, 507: image data comparison unit
405, 505 disease diagnosis reference image DB storage unit
125: lens
127: filter
129: optical fiber
235: support portion
237: ring

### [Best Mode for Embodying the Invention]

Exemplary embodiments will now be described more fully with reference to the accompanying drawings to clarify aspects, other aspects, features and advantages of the present invention, which is defined by the appended claims. The exemplary embodiments may, however, be embodied in many different forms and should not be construed as limited to the exemplary embodiments set forth herein. Rather, the exemplary embodiments are provided so that this disclosure will be thorough and complete, and will fully convey the scope of the application to those of ordinary skill in the art.

It will be understood that when an element is referred to as being "on" another element, the element can be directly on another element or intervening elements. The terms "unit" and "module" and the terms having suffix "-er" or "-or" used in the following description refer to a unit for processing at least one function or operation, and may be implemented by hardware, software, or a combination of hardware and software.

Expressions such as "transmitting," "communicating," "receiving," "providing," or "forwarding" signals, data, or information, used in the following descriptions, or other expressions similar to the aforementioned expressions may refer to directly forwarding signals, data, or information from one element ("element a") to another element ("element b"), and also refer to forwarding to element b via at least one other element ("element *c*").

In the following description, elements "operatively related to each other" should be interpreted as being connected with each other in a wired and/or wireless manner so as to transmit and/or receive data. Although there is no explicit expression "an element ("element *a*") and another element ("element *b*") are operatively related to each other" in the description, if element *a* receives signals, data, or information outputted from element *b* and performs its operation ("element *a*"), or element *b* receives signals, data, or information outputted from element *a* and performs its operation ("element *b*")*,* it should be understood that element *a* and element *b* are "operatively related to each other."

In the following description, a network may be configured by WiFi, Internet, a Local Area Network (LAN), a wireless LAN, a Wide Area Network (WAN), a Personal Area Network (PAN), 3G, 4G, LTE, a voice network, or a combination of two or more of the aforementioned networks.

The terms used herein are for the purpose of describing particular exemplary embodiments only and are not intended to limit the present invention. As used herein, the singular forms are intended to include the plural forms as well, unless the context clearly indicates otherwise. It will be further understood that the terms "comprises" and/or "comprising," when used in this specification, do not preclude the presence or addition of one or more other components.

### Definition of Terms

In the following description, the term "laser" means a pulsed laser, a continuous light laser, a focused laser, or a collimated laser. In addition, the frequency band of the "laser" may have a certain frequency band, for example, an ultra violet (UV) band, a visible light band, or an infrared (IR) band.

In addition, the term "generated light" used in the following description encompasses all types of light which are generated when a laser is projected onto body tissue. Accordingly, the "generated light" may mean plasma light, reflected light, scattered light, and/or fluorescent light.

In addition, in the following description, "an aesthetic or medical laser irradiation device" refers to a device which projects a laser and performs an operation for beauty or medical care.

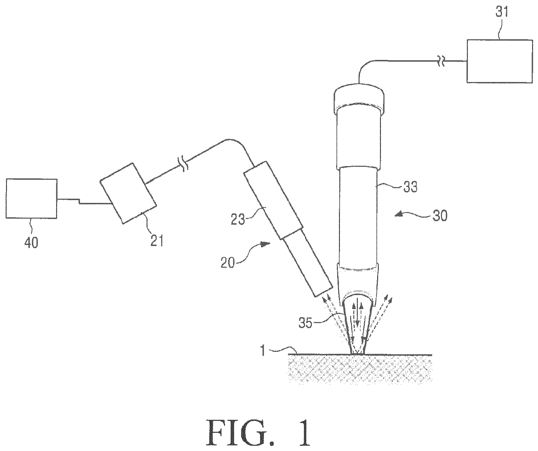

FIG. 1 is a view to illustrate a laser-based disease diagnosis system according to one embodiment of the present invention.

Referring to FIG. 1, the laser-based disease diagnosis system (hereinafter, referred to as a "disease diagnosis system") according to one embodiment of the present invention performs an operation for beauty care by irradiating body tissue with a laser for beauty or medical care, and diagnoses whether there is a disease on body tissue by collecting light generated from the body tissue and analyzing the spectrum of the generated light.

Referring to FIG 1, the disease diagnosis system according to an example illustrating the present invention may include an analysis device 20 and a laser irradiation device 30. In the present embodiment, the disease diagnosis system may further include a display 40.

In the present embodiment, the laser irradiation device 30 is a device which performs an operation for medical or beauty care with respect to body tissue using a laser, and for example, may be a skin toning device, a skin peeling device, or a laser surgery device.

In the present embodiment, the laser irradiation device 30 may irradiate body tissue 1 with a laser for the purpose of medical or beauty care.

In the present embodiment, the laser irradiation device 30 includes a handpiece 33 and a laser main body 31 for generating a laser. The laser main body 31 includes a laser generation source (not shown) for generating a laser and the laser generated by the laser generation source (not shown) is provided to the handpiece 33.

In the present embodiment, the laser irradiation device 30 may further include a guide portion. The guide portion guides the laser emitted from the handpiece 33 to be projected onto a part desired by a user. In the present embodiment, the guide portion is coupled to a distal end of the handpiece 33 and is configured to include a circular ring and a support portion 35.

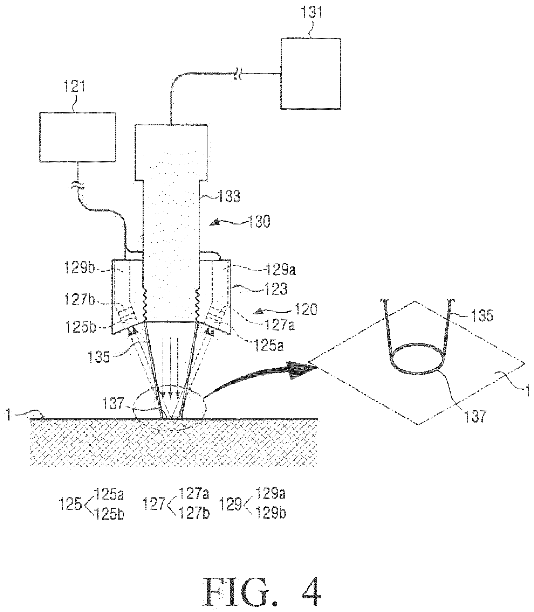

The circular ring and the support portion 35 included in the guide portion of FIG. 1 has the same configurations as a circular ring 137 and a support portion 135 of a guide portion of FIG. 4, and a description thereof is replaced with a description of the guide portion of FIG. 4.

The analysis device 20 may collect light (hereinafter, referred to as "generated light") which is generated from the body tissue 1 when the body tissue 1 is irradiated with a laser, and may diagnose a disease existing in the body tissue 1 by analyzing the spectrum ("light emission spectrum") of the collected light.

The analysis device 20 performs a function of diagnosing a disease by analyzing the spectrum of light. For example, US 7,092,087 (August 15, 2006) (hereinafter, '087' Patent) discloses a technical concept of diagnosing a disease by analyzing the spectrum of light. The feature disclosed in this patent is using the principle that a specific element emits light of a specific wavelength when a specimen is irradiated with a laser.

The analysis device 20 may also measure a skin age by analyzing the spectrum of the generated light.

When the body tissue 1 is skin, the analysis device 20 may diagnose the presence/absence of a disease and measure a skin age by analyzing the spectrum of the generated light.

The display 40 may display a result of diagnosing by the analysis device 20. The display 40 is a device which is provided with a monitor, and for example, may be a personal computer (PC) or a mobile device such as a smart phone or a personal digital assistant (PDA).

The analysis device 20 may include an analysis unit 21 and a probe 23.

In the present embodiment, the probe 23 is configured to collect the generated light from the body tissue 1, and the analysis unit 21 may determine whether there is a disease and measure a skin age by analyzing the spectrum of the generated light.

The analysis unit 21 may analyze the spectrum of the generated light collected by the probe 23, and determine whether there is a disease by comparing the analyzed spectrum and a DB for diagnosing a disease (a disease diagnosis reference spectrum data DB)-a DB in which a specific disease is matched with a light emission spectrum.

When the body tissue 1 is skin, the analysis unit 21 may also measure a skin age. That is, the analysis unit 21 may determine whether there is a disease and measure a skin age by analyzing the generated light collected by the probe 23. When measuring a skin age, the analysis unit 21 may measure the skin age by comparing to a DB for measuring a skin age (a skin age measurement reference spectrum data DB, in which a skin age is matched with a light emission spectrum). For example, the skin age measurement reference spectrum data DB may be a DB in which a spectrum of collagen is matched with a skin age.

Since the spectrum of generated light is changed due to a change in a chemical component such as a change in collagen in skin, the analysis unit 21 may measure a skin age by analyzing the spectrum of generated light.

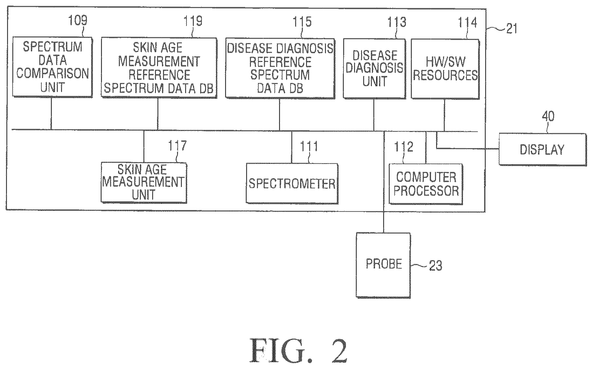

Hereinafter, the operation of the analysis unit 21 will be described in detail with reference to FIG. 2.

FIG. 2 is a view to illustrate an analysis unit according to one embodiment.

Referring to FIG. 2, the analysis unit 21 according to one embodiment of the present invention may include a spectrum data comparison unit 109, a spectrometer 111, a computer processor 212, a disease diagnosis unit 113, hardware and software (HWISW) resources 114, a disease diagnosis reference spectrum data DB storage unit 115, a skin age measurement unit 117, and a skin age measurement reference spectrum data DB storage 119.

The HW/SW resources 114 refer to hardware and software which are required for the analysis unit 21 to perform its own operation.

For example, the HW/SW resources 114 may include a memory (not shown), a storage unit (not shown) which is able to store and read data, image processing software (not shown) and hardware (not shown) for processing images to be displayed on the display 40, voice processing software (not shown) and hardware (not shown), transmitters and receivers (not shown) for transmitting and receiving data to or from the outside, and programs for operating the respective elements,

The computer processor 112 controls the elements of the analysis unit 21, for example, the spectrum data comparison unit 109, the spectrometer 111, the disease diagnosis unit 113, the HW/SW resources 114, the disease diagnosis reference spectrum data DB storage 115, the skin age measurement unit 117, and the skin age measurement reference spectrum data DB storage 119, to perform their respective operations.

The spectrometer 111 may receive generated light which is collected by the probe 23 and measures the spectrum of the generated light.

The spectrum data comparison unit 109 compares the spectrum data measured by the spectrometer 111 and the disease diagnosis reference spectrum data DB.

The disease diagnosis unit 113 determines whether there is a disease in the body tissue based on a result of comparing by the spectrum data comparison unit 109. For example, when there is the same or very similar data as or to the spectrum of the generated light in the disease diagnosis reference spectrum data DB, as a result of comparing the spectrum of the generated light measured by the spectrometer 111 and the disease diagnosis reference spectrum data DB, the disease diagnosis unit 113 may determine that there is a disease in the body tissue.

The skin age measurement reference spectrum data DB may be a DB including data having an amount of collagen and a skin age matched with each other.

The skin age measurement unit 117 may measure a skin age by comparing the spectrum measured by the spectrometer 111 and the skin age measurement reference spectrum data DB.

For example, the skin age measurement unit 117 may identify a component for measuring a skin age (for example, the component for measuring a skin age may be collagen) based on the spectrum of the generated light measured by the spectrometer 111, and may identify a skin age corresponding to an amount of identified collagen with reference to the skin age measurement reference spectrum data DB 119.

The result of diagnosing by the disease diagnosis unit 113 and the result of measuring by the skin age measurement unit 117 may be displayed on the display 40.

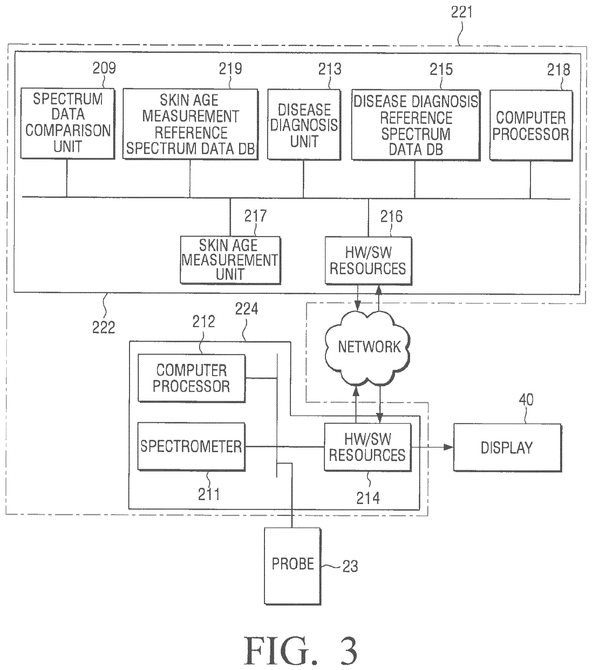

FIG. 3 is a view to illustrate an analysis unit according to another embodiment of the present disclosure.

Referring to FIG. 3, the analysis unit 221 according to another embodiment of the present invention may include a diagnosis server 222 and a diagnosis terminal 224. Herein, the diagnosis server 222 and the diagnosis terminal 224 may be operatively connected with each other via a communication network.

The diagnosis server 222 may include a spectrum data comparison unit 209, a disease diagnosis unit 213, a disease diagnosis reference spectrum data DB storage unit 215, a skin age measurement unit 217, a skin age measurement reference spectrum data DB storage unit 219, HW/SW resources 216, and a computer processor 218.

The diagnosis terminal 224 may include a spectrometer 211, a computer processor 212, and HW/SW resources 214. The diagnosis terminal 224 may transmit the spectrum of generated light which is collected by the probe 23 to the diagnosis server 222, and may receive results of diagnosing and measuring from the diagnosis server 222. The received results of diagnosing and measuring may be displayed on the display 40.

The HWISW resources 214 refer to hardware and software which are necessary for performing the operation of the diagnosis terminal 224. The HW/SW resources 214 may include a memory (not shown), a storage unit (not shown) which is able to store and read data, image processing software (not shown) and hardware (not shown) for processing images to be displayed on the display 40, voice processing software (not shown) and hardware (not shown), transmitters and receivers (not shown) for transmitting and receiving data to or from the outside, and programs for operating the respective elements.

The HW/SW resources 216 refer to hardware and software which are necessary for performing the operation of the diagnosis server 222. The HW/SW resources 214 may include a memory (not shown), a storage unit (not shown) which is able to store and read data, transmitters and receivers (not shown) for transmitting and receiving data to or from the outside, and programs for operating the respective elements.

The computer processor 212 may control the elements of the diagnosis terminal 224 , for example, the spectrometer 211 and the HW/SW resources 214, to perform their respective operations, and the computer processor 218 may control the elements of the diagnosis server 222, for example, the spectrum data comparison unit 209, the disease diagnosis unit 213, the disease diagnosis reference spectrum data DB storage unit 215, the skin age measurement unit 217, the skin age measurement reference spectrum data DB storage unit 219, and the HW/SWI resources 216, to perform their respective operations.

Referring to FIG, 3, the spectrometer 211 measures the spectrum of generated light which is collected by the probe 223, and transmits the spectrum measured by the spectrometer 211 to the diagnosis server 222 via a network.

The diagnosis server 222 may transmit results of diagnosing a disease and measuring a skin age by analyzing the spectrum received from the diagnosis terminal 224 to the diagnosis terminal 224 via the network. The result received by the diagnosis terminal 224 may be displayed by the display 40.

The operation of the diagnosis server 222 will be described in detail.

The diagnosis server 222 receives the spectrum measured by the spectrometer 211.

The spectrum data comparison unit 209 compares the disease diagnosis reference spectrum data DB and the spectrum data measured by the spectrometer 211.

The disease diagnosis unit 213 determines the presence/absence of a disease based on the result of comparing by the spectrum data comparison unit 209. In addition, the skin age measurement unit 217 may measure a skin age by comparing the received spectrum and the skin age measurement reference spectrum data DB.

The diagnosis server 222 transmits the result of diagnosing by the disease diagnosis unit 213 and the result of measuring by the skin age measurement unit 217 to the diagnosis terminal 224 via the network. Thereafter, the results transmitted to the diagnosis terminal 224 may be displayed on the display 40.

The analysis unit according to embodiments of the present invention has been described above with reference to FIGs. 2 and 3. In these embodiments, the analysis unit includes the skin age measurement unit 117, 217 and the skin age measurement reference spectrum data DB. However, the analysis unit may not include the skin age measurement unit 117, 217 and the skin age measurement reference spectrum data DB. That is, the analysis unit according to one embodiment of the present invention may not measure a skin age and may only diagnose a disease.

Referring back to FIG. 1, the other elements will be described.

The generated light collected by the probe 23 is light which is generated from the body tissue 1 when the body tissue 1 is irradiated with a laser.

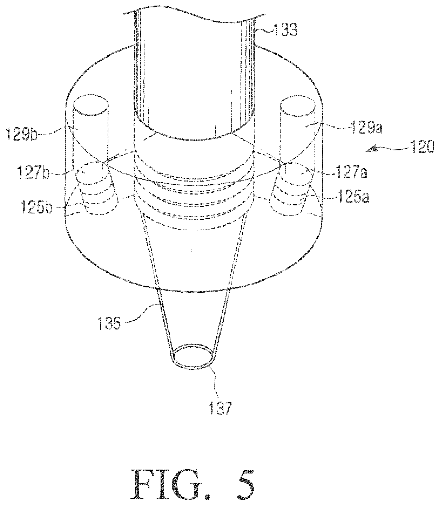

FIGs. 4 and 5 are views to illustrate a laser-based disease diagnosis system which is not covered by the claimed invention.

Referring to FIGs. 4 and 5, the laser-based disease diagnosis system includes a laser irradiation device 130 and an analysis device 120,

The analysis device 120 may include an analysis unit 121 and a probe 123. Herein, the analysis unit 121 may be the analysis unit which has been described with reference to FIGs. 1 to 3.

Compared with the embodiment of FIG. 1, the embodiment of FIG 4 differs in that the probe 123 and the laser irradiation device are removably coupled to each other.

Referring to FIGs. 4 and 5, the probe 123 is coupled to the laser irradiation device 130. For example, the probe 123 may be coupled to the laser irradiation device 130 by means of a screw in the form of enclosing the laser irradiation device 130 as shown in the drawings. Coupling by means of a screw is merely an example, and the probe 123 and the laser irradiation device 130 may be coupled to each other by other coupling methods.

In the present embodiment, the laser irradiation device 130 includes a handpiece 133 and a laser main body 131 for generating a laser.

In an embodiment, the probe 123 is formed in a substantially cylindrical shape and has a cavity formed in the center thereof. The laser irradiation device 133 may be coupled to the probe 123 by inserting the handpiece 133 into the cavity of the probe 123. This coupling method is for making it easy for the probe 123 to receive light generated by a laser projected onto the body tissue 1 through the handpiece 123.

The handpiece includes a structure for screwing and a guide portion which are formed at the distal end thereof, and the guide portion is to provide the user with convenience so as to allow the laser emitted from the handpiece 133 to be projected onto an appropriate location.

In the present embodiment, the guide portion includes a ring 137 and a support potion 135. The support portion 135 maintains the ring 137 being spaced from the main body of the handpiece 133. The support portion 135 connects the ring 137 and the distal end of the handpiece 133, but maintains the ring 137 and the distal end of the handpiece 133 being spaced from each other by a predetermined distance. The support portion 135 may be made of a material like a steel wire.

The laser irradiation device 130 is the same or similar as or to the laser irradiation device 30 described in FIG. 1 in its functions except for that the laser irradiation device 130 is coupled to the probe 123.

The analysis device 120 may collect light generated from the body tissue 1 when the body tissue 1 is irradiated with a laser, and may diagnose a disease existing in the body tissue 1 by analyzing the spectrum of the generated light and may measure a skin age when the body tissue 1 is skin.

The analysis device 120 may diagnose a disease by analyzing the spectrum of light, and for example, may perform a function of diagnosing a disease using laser induced breakdown spectroscopy (LIBS). Herein, LIBS is merely an example and other methods of spectroscopy can be applied.

When measuring a skin age, the analysis device 120 may measure the skin age by comparing to a DB (not shown) for measuring a skin age, in which a skin age is matched with a light emission spectrum.

In the present embodiment, the probe 123 collects light generated from the body tissue 1, and the analysis unit 121 may determine whether there is a disease by analyzing the generated light collected by the probe 123 and may measure a skin age when the body tissue 1 is skin. Herein, the generated light actually collected by the probe 123 is light which is generated from the body tissue 1 when the body tissue is irradiated with a laser.

Referring to FIGs. 4 and 5, the probe 123 may include a lens 125 (125a, 125b) for collecting generated light, a filter 127 (127a, 127b) for passing only a predetermined frequency band in the generated light collected by the lens 125 (125a, 125b), and an optical fiber 129 (129a, 129b) for providing a path to allow the filtered light to travel therethrough. Herein, the generated light traveling through the optical fiber 129 (129a, 129b) is provided to the spectrometer of the analysis unit 121. The

lens 125 may include a first lens 125a and a second lens 125b, the filter 127 may include a first filter 127a and a second filter 127b, and the optical fiber 129 may include a first optical fiber 129a and a second optical fiber 129b. Herein, the first lens 125a and the second lens 125b may have the same function, the first filter 127a and the second filter 127b may have the same function, and the first optical fiber 129a and the second optical fiber 129b may have the same function. In the present embodiment, two lenses, two filters, and two optical fibers are included, but this is merely an example. One lens, one filter, and one optical fiber may be included (that is, a configuration including the first lens, the first filter, and the first optical fiber) and the number of elements in each unit may be three or more.

In the system described above with reference to FIGs. 4 and 5, the probe 123 is removably coupled to the irradiation device 130. However, this is merely an example and the probe 123 may be fixed to the laser irradiation device 130. In this case, the elements 125, 127, and 129 included in the probe 123 may be arranged inside the handpiece 133.

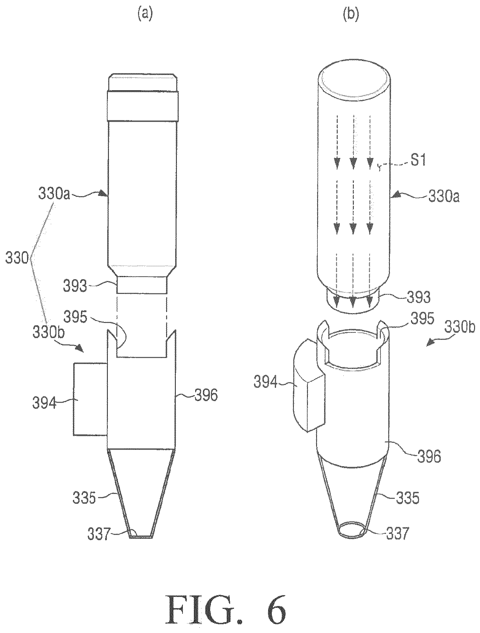

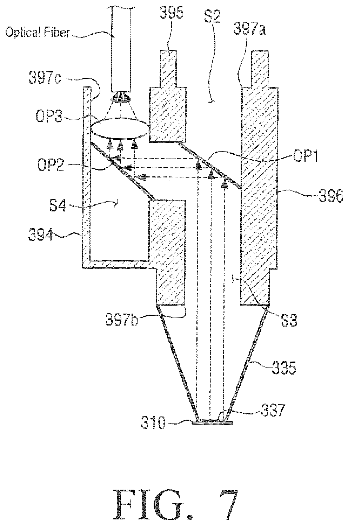

FIGs. 6 and 7 are views to illustrate a probe according to one embodiment of the present invention.

Referring to FIGs. 6 and 7, the probe according to one embodiment of the present invention is configured in the form of a handpiece detachable device such that the probe can be removably coupled to a handpiece 330a.

Referring to the drawings, the probe 330a coupled to a laser irradiation device according to one embodiment of the present invention is removably coupled to the handpiece 330a provided in the laser irradiation device to be used. Herein, the laser irradiation device is a device which irradiates body tissue with a laser and is provided with the handpiece 330a.

According to one embodiment of the present invention, the probe 330b and the handpiece 330a provided in the laser irradiation device are coupled to each other, thereby forming a handpiece 330 in the form of one piece. The handpiece 330 in the form of one piece provides using convenience to the user.

The handpiece 330 in the form of one piece in which the probe 330b and the handpiece 330a provided in the laser irradiation device are coupled to each other according to one embodiment of the present invention may be substituted for the handpiece 30 and the probe 23 described above with reference to FIG. 1.

As will be described below, an optical fiber of the handpiece 330 implemented in the form of one piece is connected to an analysis unit (for example, the analysis unit 21 described above with reference to FIGs. 1 to 3), and light provided from a laser main body (for example, the laser main body 31 described above with reference to FIG. 1) enters the handpiece 330 implemented in the form of one piece.

The probe 330b according to one embodiment of the present invention includes a body 396 having spaces S2, S3 for allowing light to travel therein. Herein, light traveling in the body 396 may be a laser provided from the laser irradiation device and light generated from body tissue.

The body 396 includes a connection portion 395, a laser entering portion 397a through which a laser is received from the laser irradiation device, a laser emission portion 397b through which the laser entering through the laser entering portion 397a is emitted to body tissue, and a generated light exit 397c through which the generated light is outputted to the outside.

Herein, the laser entering portion 397a and the laser emission portion 397b have a width and a shape enough to allow the laser to pass therethrough, and the laser entering portion 397a and the laser emission portion 397b are aligned such that the laser can pass therethrough. The generated light exit 397c may be connected with the optical fiber so as to provide the generated light to the optical fiber.

The body 396 may be formed in a cylindrical shape to allow light to travel therein, and has the connection portion 395 formed at one end of the cylindrical shape and the laser emission portion 397b formed at the other end of the cylindrical shape.

The connection portion 395 may be removably coupled to a connection portion 393 of the handpiece 330a. For example, the connection portion 395 and the connection portion 393 may have screw structures formed thereon to be fastened to each other.

In the present embodiment, the light generated from the body tissue enters the inner space S3 of the body 396 through the laser emission portion 397b. That is, the laser provided from the laser irradiation device is emitted through the laser emission portion 397b and, and the generated light from the body tissue enters the inside of the body 396 through the laser emission portion 397b.

An optical unit disposed inside the body 396 provides at least a part of the generated light entering through the laser emission portion 397b to the analysis unit (for example, the analysis unit 21 or the analysis unit 121).

In the present embodiment, the optical unit includes an optical module OP1 for changing the direction of the generated light entering through the laser emission portion 397b, an optical module OP2 for re-changing the direction of the generated light the direction of which has been changed by the optical module OP1, and an optical module OP3 for providing the generated light the direction of which has been re-changed by the optical module OP2 to the optical fiber connected with the analysis unit (for example, the analysis unit 21 or the analysis unit 121).

In an embodiment, the optical module OP1 may be an optical device which changes the direction of the generated light entering through the laser emission portion 397b by 90°, and the optical module OP2 may be an optical device which changes the direction of the generated light the direction of which has been changed by the optical module OP1 by 90°. The optical module OP3 may be an optical device which provides the generated light the direction of which has been changed by the optical module OP2 to the optical fiber like a lens.

In an embodiment, the body 396 may include a first part, a second part, and a third part, and these parts provide paths through which light travels. The first part is where the first optical module OP1 is located, the second part is where the optical modules OP2 and OP3 are located, and the third part is a guide portion.

In an embodiment, the first part may be formed in a cylindrical shape such that it can receive the laser provided from the laser irradiation device and emit the laser to the body tissue as it is. The connection portion 395 and the laser entering portion 397a may be formed at one end of the first part and the laser emission portion 397b may be formed at the other end of the first part. In embodiments, the portion given reference numeral 397 may be referred to as an "entering portion" or a "laser emission portion" in the description. This is because the light generated from the body tissue may enter through the portion given reference numeral 397 or the laser provided from the laser irradiation device may be emitted to the body tissue through the portion given reference numeral 397.

In an embodiment, the entering portion t and the laser emission portion are located at the same location, but this is merely an example, It would be easily understood by a person skilled in the art that the entering portion and the laser emission portion may be located at different locations.

In an embodiment, the laser provided from the laser irradiation device enters the first part where the laser entering portion 397a is formed, and the entering laser is projected onto the body tissue.

In an embodiment, the first part is located at the center of the body 396, and the second part is located to enclose the first part.

According to one embodiment, the probe 330b may further include a guide portion. The guide portion forms the third part as described above, and is connected with the entering portion 397 of the body 396. The guide portion includes a ring 337 and a support portion 335. The guide portion guides the laser outputted through the entering portion 397 to be projected onto a part desired by the user.

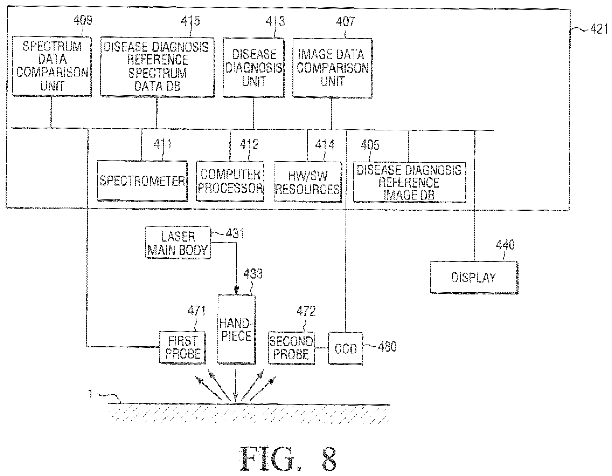

FIG. 8 is a view to illustrate a laser-based disease diagnosis system according to another embodiment.

Referring to FIG. 8, the laser-based disease diagnosis system according to one embodiment of the present invention may perform an operation for beauty or medical care by irradiating body tissue with a laser using the laser, and may diagnose a disease in the body tissue by collecting light generated from the body tissue, collecting an image regarding a part irradiated with the laser, and analyzing the spectrum of the generated light and the collected image.

Referring to FIG. 8, the present system may include an analysis device and a laser irradiation device. The present system may further include a display 440.

Referring to FIG. 8, the analysis device may include an analysis unit 421, a first probe 471, a second probe 472, and a CCD 480, and the laser irradiation device may include a laser main body 431 and a handpiece 433. Herein, the analysis unit 421 may include an image data comparison unit 407, a spectrum data comparison unit 409, a spectrometer 411, a computer processor 412, a disease diagnosis unit 413, HWISW resources 414, a disease diagnosis reference spectrum data DB storage unit 415, and a disease diagnosis reference image DB storage unit 421.

According to the present embodiment, the laser main body 431 generates a laser and the handpiece 422 irradiates body tissue 1 with the laser. When the body tissue 1 is irradiated with the laser, light is generated from the body tissue 1.

The first probe 471 is disposed to easily collect the generated light from the body tissue 1. For example, the first probe 471 may be permanently attached or removably attached to the handpiece 433 or may not be physically coupled to the handpiece 433. The first probe 471 may be configured to include one or more optical elements (for example, a lens or an optical fiber for providing a moving path of light) so as to easily collect the generated light from the body tissue 1. The generated light collected by the first probe 471 is provided to the spectrometer 411.

The second probe 472 and the CCD (charged coupled device) (hereinafter, "CCD") 480 are devices for collecting an image (hereinafter, an "analysis target image") regarding the body tissue 1. The second probe 472 includes optical elements for allowing the CCD 480 to easily collect the analysis target image. In the present specification, the second probe 472 and the CCD 480 are referred to as an "image collection unit" for easy explanation of the present invention.

The image collection unit is disposed to easily collect the analysis target image. The image collection unit may be permanently attached or removably attached to the handpiece 433, and may not be physically coupled to the handpiece 433. In addition, the image collection unit may be physically coupled to the first probe 471. The analysis target image collected by the image collection unit is provided to the image data comparison unit 407.

The second probe 472 may be configured to include one or more optical elements (for example, a lens or an optical fiber for providing a moving path of light) for allowing the CCD 480 to easily collect the analysis target image.

The image collection unit necessarily includes a device for photographing an image like the CCD 480, but may not necessarily include the second probe 472. For example, when the CCD 480 is disposed to easily collect the analysis target image, the second probe 472 may not be used.

Regarding exemplary configurations and operations of the first probe and the second probe, please refer to Korean Patent Registration No. 10-1640202 (July 11, 2016) since Korean Paten Registration No. 10-1640202 (July 11, 2016) discloses technology of determining the presence/absence of a disease using a spectrum of generated light.

Operations of the spectrum data comparison unit 409, the spectrometer 411, the computer processor 412, the HW/SW resources 414, and the disease diagnosis reference spectrum data DB storage unit 415 are the same as the operations of the spectrum data comparison unit 409, the spectrometer 111, the computer processor 112, the HW/SW resources 114, and the disease diagnosis reference spectrum data DB storage unit 115 described above with reference to FIG. 2, respectively, and thus a description thereof is omitted.

The image data comparison unit 407 compares the analysis target image collected by the image collection unit and a disease diagnosis reference image DB stored in the storage 421, and provides a result of comparing to the disease diagnosis unit 413.

The disease diagnosis unit 413 may determine whether there is a disease in the body tissue 1 by analyzing at least one of the result of comparing by the spectrum data comparison unit 409 and the result of comparing by the image data comparison unit 407.

In one example of the determination method, i) when there exists the same or very similar data as or to the spectrum data of the generated light in the disease diagnosis reference spectrum data DB, or ii) when there exists the same or very similar image as or to the analysis target image in the disease diagnosis image DB, the disease diagnosis unit 413 may determine that there exists a disease in the body tissue 1.

In another example of the determination method, i) when there exists the same or very similar data as or to the spectrum data of the generated light in the disease diagnosis reference spectrum data DB, and ii) when there exists the same or very similar image as or to the analysis target image in the disease diagnosis image DB, the disease diagnosis unit 413 may determine that there exists a disease in the body tissue 1.

Description of the elements of FIG. 8 which have not been described is replaced with the description of FIG. 2.

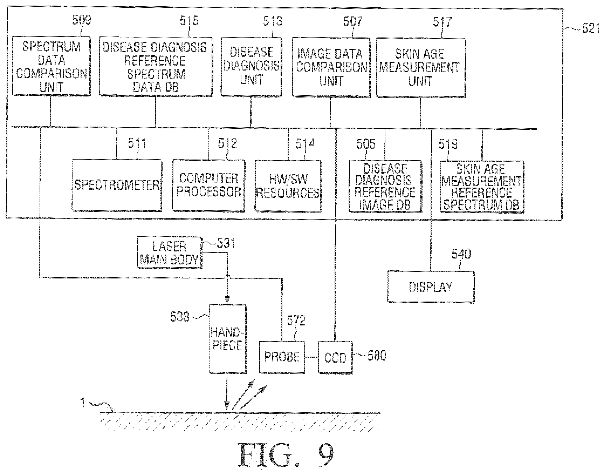

FIG. 9 is a view to illustrate a laser-based disease diagnosis system according to another embodiment.

Referring to FIG. 9, the present system performs operations for beauty or medical care by irradiating body tissue with a laser using the laser, and may diagnose whether there exists a disease in the body tissue and may measure an age of body tissue by collecting light generated from the body tissue, collecting an image regarding a part irradiated with the laser, and analyzing the spectrum of the generated light and the collected image.

Referring to FIG. 9, the present system may include an analysis device and a laser irradiation device. The present system may further include a display 540.

Referring to FIG. 9, the analysis device includes an analysis unit 521, a probe 572, and a CCD 580, and the laser irradiation device includes a laser main body 531 and a handpiece 533. Herein, the analysis unit 521 may include an image data comparison unit 507, a spectrum data comparison unit 509, a spectrometer 511, a computer processor 512, a disease diagnosis unit 513, HW/SW resources 514, a disease diagnosis reference spectrum data DB storage unit 515, a skin age measurement unit 517, a skin age measurement reference spectrum DB 519, and a disease diagnosis reference image DB storage 521.

The embodiment described with reference to FIG. 8 and the embodiment described with reference to FIG. 9 differ from each other in that a skin age is measured in the embodiment of FIG. 9. Hereinafter, the difference will be mainly described.

According to the embodiment of FIG. 9, the laser main body 531 generates a laser, and the handpiece 533 irradiates body tissue 1 with the laser generated by the laser main body 531. When the body tissue 1 is irradiated with the laser, light is generated from the body tissue 1.

The probe 572 includes one or more optical elements to collect the light generated from the body tissue 1 and to easily provide an analysis target image to the CCD 580. Herein, the optical element may function to reflect, refract, or pass the entirety or part of light and/or an image.

The probe 572 may be permanently attached or removably attached to the handpiece 533, or may not be physically coupled to the handpiece 533.

The generated light collected by the probe 572 is provided to the spectrometer 511 and the analysis target image is converted into an image by the CCD 580 and provided to the image data comparison unit 507.

The operation of the disease diagnosis unit 513 is the same as the operation of the disease diagnosis unit 413 described above with reference to FIG. 8, and thus a detailed description thereof is omitted.

The skin age measurement reference spectrum data DB may be a DB including data having an amount of collagen and a skin age matched with each other.

The skin age measurement unit 517 may measure a skin age from the spectrum measured by the spectrometer 511 with reference to the skin age measurement reference spectrum data DB 519.

For example, the skin age measurement unit 517 may identify a component (for example, the component for measuring a skin age may be collagen) for measuring a skin age based on the spectrum of the generated light measured by the spectrometer 511, and may identify a skin age corresponding to an amount of identified collagen with reference to the skin age measurement reference spectrum data DB 519.

A detailed description of the skin age measurement unit 517 may be replaced with the description of the skin age measurement unit 117 described above with reference to FIG. 2.

While the invention has been shown and described with reference to certain preferred embodiments thereof and the drawings, the present invention is not limited by the above-described embodiments. It will be understood by those skilled in the art that various changes in form and details may be made therein without departing from the scope of the invention as defined by the appended claims. Therefore, the scope of the invention is defined not by the detailed description of the invention but by the appended claims .

## Claims

1. A laser-based disease diagnosis system comprising:
a laser irradiation device (30, 130) configured to project a laser onto a body tissue, wherein the laser irradiation device (30, 130) further comprises a laser main body (31, 431, 531) configured to generate the laser and a handpiece (33, 133, 330a, 433, 533) configured to provide a traveling path from the laser main body to outside of the laser irradiation device for the laser generated by the laser main body;
a probe (23, 123, 330b, 471, 572) detachably coupled to an outside of the handpiece and configured to collect light generated from the body tissue after the laser is projected onto the body tissue, wherein the light is plasma light;
a support portion (35, 135, 335) formed extended from the probe so as to be able to maintain a distance from the body tissue to the laser irradiation device;
a ring (35, 137, 337) formed at an end of the support portion and configured to contact the body tissue around at least a portion of the body tissue;
a spectrometer (111, 211, 411, 511) configured to measure a spectrum data of the light collected by the probe;
a spectrum data comparison unit (109, 209, 409) electrically connected to the spectrometer and configured to compare the spectrum data of the plasma light and a reference spectrum data; and
a disease diagnosis unit (113, 213, 413) configured to determine whether there is a disease in the body tissue based on the comparison result of the spectrum data of the plasma light and the reference spectrum data,
wherein the probe further comprises an optical structure comprising:
a body (396) including a first hole (S2) to allow the laser to travel therein and a second hole (S4) formed to face a different direction from the first hole,
a laser emission portion (397b) disposed on an end of the body and including a third hole (S3) optically connected to the first hole, wherein the third hole is connected to an outside of the body such that the plasma light is able to enter the body, and a cavity from the third hole to the first hole provides a common optical path for both the laser generated by the laser main body and the plasma light generated from the body tissue,
a first optical element (OP1) disposed between the first hole and the third hole and configured to reflect the plasma light but to transmit the laser so that a traveling direction of the laser is maintained, wherein a reflective surface of the first optical element is arranged to reflect the plasma light towards the second hole which is formed to be branched from the cavity,
a second optical element (OP2) disposed parallel to the first optical element and configured to reflect the plasma light reflected from the first optical element,
a third optical element (OP3) disposed adjacent to the second optical element and configured to focus the plasma light reflected from the second optical element, and
an optical fiber (129) optically connected to the third optical element and configured to transmit the plasma light focused by the third optical element to the spectrometer.

2. The laser-based disease diagnosis system of claim 1, wherein the laser is a focused laser.

3. The laser-based disease diagnosis system of claim 1, wherein the probe further collects a fluorescent light.

## Patentansprüche

1. Laserbasiertes Krankheitsdiagnosesystem, umfassend:
eine Laserbestrahlungsvorrichtung (30, 130), ausgebildet zum Projizieren eines Lasers auf ein Körpergewebe, wobei die Laserbestrahlungsvorrichtung (30, 130) ferner einen Laserhauptkörper (31, 431, 531), ausgebildet zum Erzeugen des Lasers, und ein Handstück (33, 133, 330a, 433, 533), ausgebildet zum Bereitstellen eines Bewegungsweges vom Laserhauptkörper zur Außenseite der Laserbestrahlungsvorrichtung für den vom Laserhauptkörper erzeugten Laser, umfasst;
eine Sonde (23, 123, 330b, 471, 572), abnehmbar an eine Außenseite des Handstücks gekoppelt und ausgebildet zum Sammeln von vom Körpergewebe erzeugtem Licht, nachdem der Laser auf das Körpergewebe projiziert wurde, wobei das Licht Plasmalicht ist;
einen Stützabschnitt (35, 135, 335), sich von der Sonde erstreckend gebildet, so dass er einen Abstand vom Körpergewebe zur Laserbestrahlungsvorrichtung halten kann;
einen Ring (35, 137, 337), gebildet an einem Ende des Stützabschnitts und ausgebildet zum Berühren des Körpergewebes um wenigstens einen Abschnitt des Körpergewebes;
ein Spektrometer (111, 211, 411, 511), ausgebildet zum Messen von Spektrumsdaten des von der Sonde gesammelten Lichts;
eine Spektrumsdaten-Vergleichseinheit (109, 209, 409), elektrisch mit dem Spektrometer verbunden und ausgebildet zum Vergleichen der Spektrumsdaten des Plasmalichts und von Referenzspektrumsdaten; und
eine Krankheitsdiagnoseeinheit (113, 213, 413), ausgebildet zum Bestimmen, ob eine Krankheit im Körpergewebe vorliegt, auf Basis des Vergleichsergebnisses der Spektrumsdaten des Plasmalichts und der Referenzspektrumsdaten,
wobei die Sonde ferner eine optische Struktur umfasst, umfassend:
einen Körper (396), umfassend ein erstes Loch (S2), um dem Laser ein Bewegen darin zu ermöglichen, und ein zweites Loch (S4), gebildet zum Zeigen zu einer anderen Richtung vom ersten Loch,
einen Laseremissionsabschnitt (397b), angeordnet an einem Ende des Körpers und umfassend ein optisch mit dem ersten Loch verbundenes drittes Loch (S3), wobei das dritte Loch mit einer Außenseite des Körpers verbunden ist, so dass das Plasmalicht in den Körper eindringen kann, und ein Hohlraum vom dritten Loch zum ersten Loch einen gemeinsamen optischen Weg für sowohl den vom Laserhauptkörper erzeugten Laser als auch das vom Körpergewebe erzeugte Plasmalicht bereitstellt,
ein erstes optisches Element (OP1), angeordnet zwischen dem ersten Loch und dem dritten Loch und ausgebildet zum Reflektieren des Plasmalichts, aber zum Übertragen des Lasers, so dass eine Bewegungsrichtung des Lasers beibehalten wird, wobei eine Reflexionsfläche des ersten optischen Elements zum Reflektieren des Plasmalichts zum zweiten Loch, das zum Abzweigen vom Hohlraum gebildet ist, angeordnet ist,
ein zweites optisches Element (OP2), angeordnet parallel zum ersten optischen Element und ausgebildet zum Reflektieren des vom ersten optischen Element reflektierten Plasmalichts,
ein drittes optisches Element (OP3), angeordnet angrenzend zum zweiten optischen Element und ausgebildet zum Fokussieren des vom zweiten optischen Element reflektierten Plasmalichts, und
eine optische Faser (129), optisch mit dem dritten optischen Element verbunden und ausgebildet zum Übertragen des vom dritten optischen Element fokussierten Plasmalichts zum Spektrometer.

2. Laserbasiertes Krankheitsdiagnosesystem nach Anspruch 1, wobei der Laser ein fokussierter Laser ist.

3. Laserbasiertes Krankheitsdiagnosesystem nach Anspruch 1, wobei die Sonde ferner ein Fluoreszenzlicht sammelt.

## Revendications

1. Système de diagnostic de maladie à base de laser comprenant :
un dispositif d'irradiation laser (30, 130) configuré pour projeter un laser sur un tissu corporel, le dispositif d'irradiation laser (30, 130) comprenant en outre un corps principal de laser (31, 431, 531) configuré pour générer le laser et une pièce à main (33, 133, 330a, 433, 533) configurée pour fournir un trajet de déplacement depuis le corps principal de laser vers l'extérieur du dispositif d'irradiation laser pour le laser généré par le corps principal de laser ;
une sonde (23, 123, 330b, 471, 572) accouplée de manière détachable à un extérieur de la pièce à main et configurée pour collecter la lumière générée du tissu corporel après que le laser est projeté sur le tissu corporel, la lumière étant de la lumière plasma ;
une portion de support (35, 135, 335) formée étendue depuis la sonde afin de pouvoir maintenir une distance depuis le tissu corporel vers le dispositif d'irradiation laser ;
une bague (35, 137, 337) formée à une extrémité de la portion de support et configurée pour entrer en contact avec le tissu corporel autour d'au moins une portion du tissu corporel ;
un spectromètre (111, 211, 411, 511) configuré pour mesurer des données de spectre de la lumière collectée par la sonde ;
une unité de comparaison de données de spectre (109, 209, 409) électriquement connectée au spectromètre et configurée pour comparer les données de spectre de la lumière plasma et des données de spectre de référence ; et
une unité de diagnostic de maladie (113, 213, 413) configurée pour déterminer s'il existe une maladie dans le tissu corporel sur la base du résultat de comparaison des données de spectre de la lumière plasma et des données de spectre de référence,
la sonde comprenant en outre une structure optique comprenant :
un corps (396) incluant un premier trou (S2) pour permettre au laser de se déplacer à l'intérieur et un second trou (S4) formé pour faire face à un sens différent du premier trou,
une portion d'émission de laser (397b) disposée sur une extrémité du corps et incluant un troisième trou (S3) optiquement connecté au premier trou, le troisième trou étant connecté à un extérieur du corps de sorte que la lumière plasma peut pénétrer le corps, et une cavité depuis le troisième trou vers le premier trou fournissant un trajet optique commun pour à la fois le laser généré par le corps principal de laser et la lumière plasma générée depuis le tissu corporel,
un premier élément optique (OP1) disposé entre le premier trou et le troisième trou et configuré pour refléter la lumière plasma mais pour transmettre le laser afin qu'un sens de déplacement du laser soit maintenu, une surface de réflexion du premier élément optique étant agencée pour refléter la lumière plasma vers le second trou qui est formé pour être ramifié depuis la cavité,
un second élément optique (OP2) disposé parallèle au premier élément optique et configuré pour refléter la lumière plasma reflétée depuis le premier élément optique,
un troisième élément optique (OP3) disposé adjacent au second élément optique et configuré pour focaliser la lumière plasma reflétée du second élément optique, et
une fibre optique (129) optiquement connectée au troisième élément optique et configurée pour transmettre la lumière plasma focalisée par le troisième élément optique vers le spectromètre.

2. Système de diagnostic de maladie à base de laser selon la revendication 1, le laser étant un laser focalisé.

3. Système de diagnostic de maladie à base de laser selon la revendication 1, la sonde collectant en outre une lumière fluorescente.
